# EUROPÄISCHE PATENTSCHRIFT

(11) **EP 0 657 148 B1**
(45) Veröffentlichungstag und Bekanntmachung des Hinweises auf die Patenterteilung: **15.07.1998**
(21) Anmeldenummer: 94102444.0
(22) Anmeldetag: 17.02.1994
(51) Int. Cl.: A61F 2/52

(54) **Brustprothese zum Tragen im Oberteil eines Badebekleidungsstücks**
Breast prosthesis to be worn in the upper part of a bathing suit
Prothèse mammaire à porter dans la partie supérieure d'un costume de bain

(30) Priorität: 08.12.1993 DE 9318842 U
(43) Veröffentlichungstag der Anmeldung: 14.06.1995
(73) Patentinhaber: F + E Gesellschaft für Bekleidungsinnovation mbH & Co. KG, 83098 Brannenburg (DE)
(72) Erfinder: Weber-Unger, Georg, A-6330 Kufstein (AT)
(74) Vertreter: Haug, Dietmar, Dipl.-Ing.

(56) Entgegenhaltungen:
- EP-A- 0 465 816
- DE-A- 2 912 120
- DE-U- 1 739 612
- DE-U- 9 318 842
- US-A- 4 172 298

## Beschreibung

Die Erfindung betrifft eine Brustprothese zum Tragen im Oberteil eines Badebekleidungsstücks, die einen weich-elastischen, schalenförmigen Kunststoffkörper, der von einer Polyurethanfolie umhüllt ist, aufweist, wobei die dem Körper der Trägerin zugewandte Rückseite des Kunststoffkörpers eine Aushöhlung und mindestens eine aus der Oberfläche der Aushöhlung vorspringende Versteifungsrippe aufweist.

Eine solche Brustprothese ist aus der DE 29 12 120 A1 bekannt. Bei dieser Brustprothese, die eine kreisrunde Grundfläche hat, sind drei, in gleichen Winkelabständen von 120° angeordnete, in radialer Richtung sich erstreckende Versteifungsrippen auf der Rückseite des die Prothese bildenden Kunststoffkörpers vorgesehen. Die Rippen beginnen am äußeren Rand der Prothese und enden im radialen Abstand vom Zentrum der Aushöhlung. Infolgedessen bildet die Aushöhlung eine einzige Kammer. Der geradlinig verlaufende Rücken einer jeden Versteifungsrippe springt wulstförmig von der ringförmigen Randfläche vor, die zwischen der Aushöhlung und der Vorderseite der Prothese gebildet ist. Bei Verwendung einer solchen Prothese im Oberteil eines Badeanzugs oder eines Bikinis empfindet es die Trägerin als unangenehm, daß das in der Aushöhlung sich gesammelte Wasser nicht schnell genug aus der Aushöhlung abläuft, denn die radial inneren Enden der Versteifungsrippen bilden eine Engstelle, welche die Abströmung des Wassers behindert. Außerdem sind die zwischen der ringförmigen Randfläche und dem Körper der Trägerin, im Bereich der am Körper der Trägerin anliegenden Rücken der Versteifungsrippen gebildeten Spalte zu klein, um einen raschen Abfluß des Wassers aus der Aushöhlung zu gestatten. Ferner sind nur die beim Tragen der Prothese im unteren Bereich gebildeten Spalte für das unter der Wirkung der Schwerkraft ausströmende Wasser wirksam. Infolge der über den Prothesenrand nach hinten vorspringenden Rücken der Versteifungsrippen steht der Prothesenrand nahezu rundum am Körper der Trägerin ab. Aus ästhetischen Gründen betrachten es viele Trägerinnen aber als Nachteil, wenn der Prothesenrand nicht weitgehend und vor allem nicht im oberen Bereich der Prothese dicht am Körper der Trägerin anliegt.

Aus der DE-U-1 739 612 ist eine Brustprothese bekannt, die auf der Rückseite des Prothesenkörpers eine Aushöhlung aufweist, aus der vier kreuzförmig angeordnete Versteifungsrippen vorspringen, von denen zwei horizontal verlaufen und die beiden anderen Versteifungsrippen vertikal verlaufen, wobei alle vier Rippen in der Mitte miteinander verbunden sind. Der gesamte Prothesenkörper besteht aus einem porösen Werkstoff, der die Eigenschaft hat, zum einen den Schweiß von der Haut abzuführen, und zum anderen eine ausreichende Adhäsion am Körper zu gewährleisten. Diese Brustprothese ist als Schwimmprothese gänzlich ungeeignet, da die Versteifungsrippen das Ausströmen von Wasser aus den oberhalb der horizontalen Rippen liegenden Kammern der Aushöhlung erheblich behindern würden und sich der Prothesenkörper aufgrund der Porosität seines Werkstoffes mit Wasser vollsaugen würde. Außerdem würde das Ausströmen von Wasser aus der rückwärtigen Aushöhlung auch durch die Adhäsion der Prothese am Körper der Trägerin behindert werden.

Aus der EP-A-465 816 ist eine Brustprothese bekannt, die einen weich-elastischen, schalenförmigen Kunststoffkörper aufweist, der von einer Polyurethanfolie umhüllt ist. Auf der Rückseite des Kunststoffkörpers befindet sich eine einzige Aushöhlung, aus der keine Versteifungsrippen vorspringen. Diese Brustprothese hat eine projizierte Grundfläche, die annähernd die Form eines gleichschenkligen Dreiecks hat, dessen Ecken abgerundet sind und dessen Basis konvex gekrümmt ist, wobei die Schenkel des Dreiecks einen Winkel von ungefähr 80° einschließen. Würde diese Brustprothese beim Schwimmen getragen werden, könnte das sich in der Aushöhlung ansammelnde Wasser schlecht wieder ausströmen, denn die Prothese würde sich aufgrund ihrer geringen Steifigkeit bei Druck auf die Vorderseite der Prothese stark verformen und an den Körper der Trägerin ansaugen, so daß der Prothesenrand das in der Aushöhlung angesammelte Wasser wie eine Dichtung am Ausströmen hindern würde.

Die Aufgabe der Erfindung besteht darin, die eingangs beschriebene Brustprothese so auszubilden, daß beim Schwimmen in der Aushöhlung der Prothese gesammeltes Wasser sowohl beim Herausgehen aus dem Wasser als auch beim Schwimmen rasch ausströmen kann, und daß der Prothesenrand jedenfalls im oberen Bereich der Prothese dicht am Körper der Trägerin anliegen kann.

Die Aufgabe der Erfindung wird durch eine Brustprothese gemäß Patentanspruch 1 oder eine Brustprothese gemäß Patentanspruch 7 gelöst. Vorteilhafte Ausgestaltungen der Erfindung sind in den abhängigen Ansprüchen angegeben.

Bei der erfindungsgemäßen Brustprothese ist die Versteifungsrippe oder sind die Versteifungsrippen strömungsgünstig derart ausgebildet und angeordnet, daß beim Schwimmen der Strömungswiderstand des dem Körper der Trägerin zugewandten hinteren Teils der Prothese herabgesetzt ist und auch beim Herausgehen aus dem Wasser das in der Aushöhlung gesammelte Wasser zügig nach unten aus der Aushöhlung der Prothese abfließen kann, wobei die Versteifungsrippe oder -rippen der Prothese eine ausreichende Steifigkeit verleihen, so daß sie trotz des erhöhten Drucks des Oberteils des Badebekleidungsstücks ihre Form beibehält und durch das Oberteil des Badebekleidungsstücks nicht zusammengedrückt werden kann.

Vorzugsweise hat die oder jede Versteifungsrippe einen in Längsrichtung verlaufenden Rücken, der am oberen Ende der jeweiligen Rippe tiefer als der obere Rand der Prothese ist. Hierdurch wird die von den Trägerinnen gewünschte dichte Anlage des oberen Prothesenrandes am Körper der Trägerinnen sichergestellt. Bei einem Ausführungsbeispiel springt der Rücken am unteren Ende der jeweiligen Rippe über den unteren Rand der Prothese in Richtung des Körpers der Trägerin vor. Dadurch ergibt sich am unteren Prothesenrand ein Spalt zwischen dem unteren Prothesenrand und dem Körper der Trägerin, durch den das in der Aushöhlung der Prothese sich gesammelte Wasser nach unten zügig abströmen kann. Vorzugsweise ist der Rücken der oder jeder Versteifungsrippe auch im mittleren Bereich der jeweiligen Rippe tiefer als der obere und der seitliche Rand der Prothese. Dadurch wird sichergestellt, daß nicht nur der obere sondern auch der seitliche Prothesenrand am Körper der Trägerin dicht anliegt und ein Spalt nur zwischen dem unteren Prothesenrand und dem Körper der Trägerin gebildet ist.

Besonders bei größeren Prothesen kann die Steifigkeit der Prothese unter Beibehaltung der strömungsgünstigen Ausbildung des hinteren Teils der Prothese dadurch verbessert werden, daß mehrere geradlinig und ununterbrochen zwischen dem oberen und unteren Rand der Aushöhlung sich erstreckende Versteifungsrippen vorgesehen sind, die parallel zueinander verlaufen.

Bei einer symmetrischen Prothese, die bezüglich einer rechtwinklig auf ihrer Grundfläche stehenden Mittelebene symmetrisch ist, die beim Tragen der Prothese parallel zur Längsrichtung des Körpers der Trägerin verläuft, ist es im Hinblick auf eine strömungsgünstige Ausbildung des hinteren Teils der Prothese und eine hohe Steifigkeit der Prothese von Vorteil, wenn die oder eine der Versteifungsrippen auf der Mittelebene der Prothese verläuft.

Besonders bei größeren Prothesen ist es von Vorteil, wenn drei parallele Versteifungsrippen vorgesehen sind, von denen die mittlere auf der Mittelebene der Prothese verläuft, die bezüglich der Mittelebene symmetrisch ist.

Ein besonders hoher Tragekomfort wird dann erreicht, wenn die Grundfläche der Prothese annähernd die Form eines gleichschenkligen Dreiecks hat, dessen Ecken abgerundet sind und dessen Basis konvex gekrümmt ist und dessen Schenkel einen Winkel von 700 bis 90°, vorzugsweise 80°, einschließen und die oder eine der Versteifungsrippen auf der Höhenlinie des Dreiecks verläuft.

Vorzugsweise hat die Versteifungsrippe einen in Längsrichtung der Rippe sich erstreckenden Rücken, der in einem mittleren Längsabschnitt der Rippe konkav gebogen ist. Dadurch wird eine Anlage des mittleren Längsabschnitts der Rippe an der Operationsnarbe vermieden. Wenn mehrere Versteifungsrippen vorgesehen sind, ist vorzugsweise der Rücken einer jeden Versteifungsrippe in einem mittleren Längsabschnitt der Rippe konkav gebogen.

Ein Ausführungsbeispiel der Erfindung ist in den Zeichnungen dargestellt und wird im folgenden näher beschrieben. Es zeigt
- Figur 1: eine Draufsicht auf die Rückseite einer erfindungsgemäßen Brustprothese,
- Figur 2: eine Draufsicht auf die Vorderseite der in Figur 1 dargestellten Brustprothese,
- Figur 3: einen Längsschnitt durch die in Figur 1 dargestellte Brustprothese längs der Linie III-III der Figur 1 und
- Figur 4: einen Querschnitt der in Figur 1 dargestellten Brustprothese längs der Linie IV-IV der Figur 1.

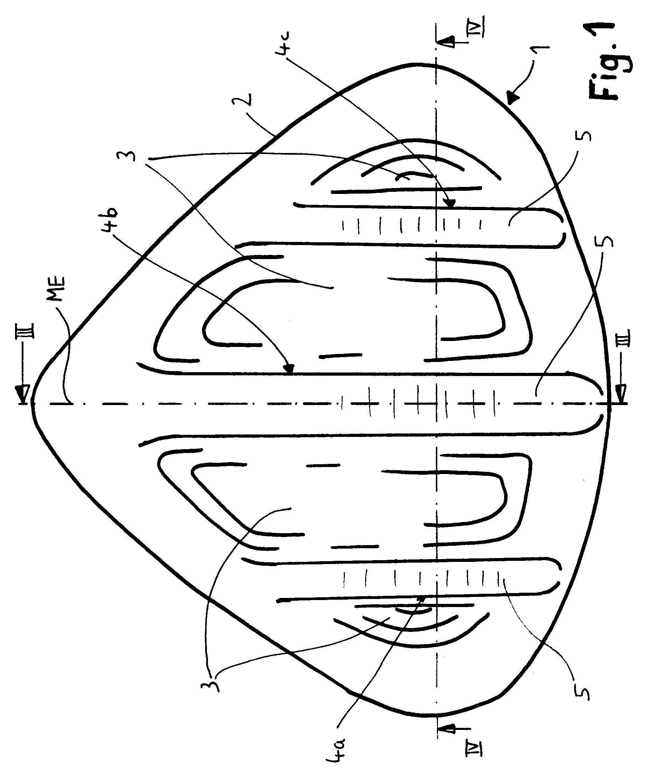

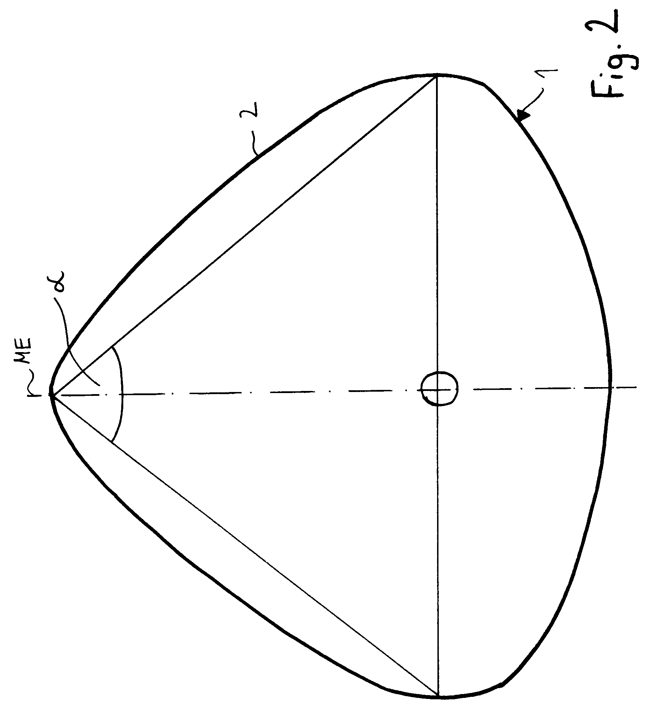

Die in den Zeichnungen dargestellte Brustprothese eignet sich besonders zum Tragen im Oberteil eines Badebekleidungsstücks und wird deshalb auch als Schwimmprothese bezeichnet. Sie besteht aus einem weich-elastischen, schalenförmigen Kunststoffkörper 1, der im wesentlichen aus einer additionsvernetzten Zweikomponenten-Silikonkautschukmasse besteht, die von einer Polyurethanfolie umhüllt ist, die aus zwei die Vorder- und Rückseite der Prothese bedeckenden Folienteilen besteht, die längs des Prothesenrandes 2 miteinander verschweißt sind. Die in Figur 2 dargestellte Vorderseite der Prothese hat eine der natürlichen Brust entsprechenden konvexe Form, während die in Figur 1 dargestellte Rückseite der Prothese eine Aushöhlung 3 aufweist, aus deren Oberfläche drei parallel verlaufende Versteifungsrippen 4a, 4b und 4c nach hinten, d.h. in Richtung des Körpers der Trägerin der Prothese vorspringen.

Wie besonders aus den Figuren 1 und 2 ersichtlich ist, hat die in die Zeichenebene projizierte Grundfläche der Brustprothese annähernd die Form eines gleichschenkligen Dreiecks, dessen Ecken abgerundet sind und dessen Basis konvex gekrümmt ist und dessen Schenkel einen Winkel α von etwa 80° einschließen. Die Prothese ist bezüglich einer rechtwinklig auf ihrer Grundfläche stehenden Mittelebene ME symmetrisch. Die Prothese wird im Oberteil eines Badeanzugs oder Bikinis so getragen, daß die Mittelebene ME parallel zur Längsrichtung des Körpers der Trägerin verläuft, wobei der Winkel α sich am oberen Ende der Prothese befindet, wobei sich "oben" auf den Tragezustand bei aufrechter Körperhaltung der Trägerin bezieht.

Alle drei Versteifungsrippen 4a, 4b, 4c erstrecken sich geradlinig und ununterbrochen zwischen dem oberen und unteren Rand der Aushöhlung 3, wobei die mittlere Versteifungsrippe 4b auf der Mittelebene ME der Prothese verläuft und die beiden seitlichen Versteifungsrippen 4a und 4c den jeweils gleichen Seitenabstand von der mittleren Versteifungsrippe 4b haben.

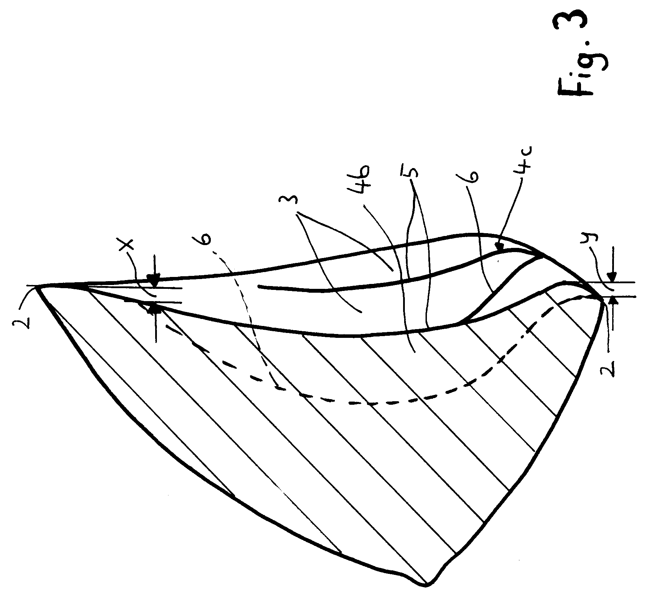

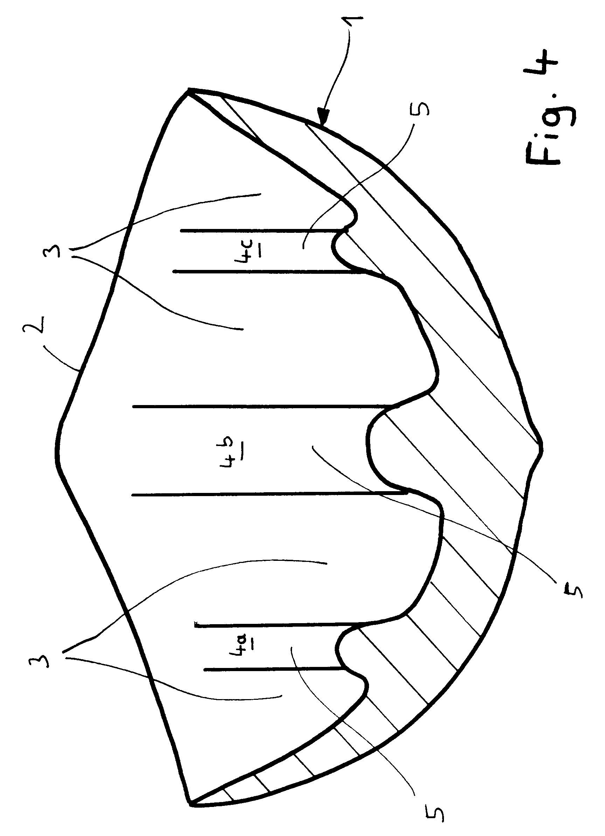

Jede Versteifungsrippe 4a, 4b, 4c hat einen, in der Draufsicht gemäß Figur 1 gesehen, geradlinig verlaufenden Rücken 5, der von einer Linie der höchsten Erhebung vom Boden 6 der Aushöhlung 3 gemessen begrenzt wird. Wie aus Figur 4 ersichtlich, hat der Rücken 5 einer jeden Versteifungsrippe 4a, 4b und 4c im Querschnitt eine konvex gebogene Form. Wie aus Figur 3 ersichtlich, ist der Rücken 5 einer jeden Versteifungsrippe 4a, 4b und 4c in einem mittleren Längsabschnitt der jeweiligen Rippe konkav gebogen, wodurch sich beim Tragen der Prothese ein Abstand zwischen dem Rücken der jeweiligen Versteifungsrippe 4a, 4b, 4c in dem mittleren Längsabschnitt der Rippe und dem Körper der Trägerin ergibt.

Wie ferner aus Figur 4 ersichtlich, enden die Versteifungsrippen 4a, 4b und 4c unterhalb des oberen Bereichs des Prothesenrandes 2, und ist ihr oberes Ende tiefer, beispielsweise um die in Figur 3 eingezeichnete Strecke X als der obere Bereich des Prothesenrandes 2 angeordnet. Auch im mittleren Bereich einer jeden Versteifungsrippe 4a, 4b, 4c liegt der Rücken 5 tiefer als der obere und auch der seitliche Bereich des Prothesenrandes 2 der Prothese. Am unteren Ende einer jeden Versteifungsrippe 4a, 4b, 4c springt der Rücken 5 über den unteren Bereich des Prothesenrandes 2 in Richtung des Körpers der Trägerin vor, beispielsweise um die in Figur 3 eingezeichnete Strecke Y. Infolgedessen liegt die Prothese in seitlichen und oberen Bereich des Prothesenrandes 2 dicht am Körper der Trägerin an, während der untere Bereich des Prothesenrandes 2 einen Abstand vom Körper der Trägerin hat, so daß dort beim Tragen der Prothese ein Spalt gebildet wird, durch den das in der Aushöhlung 3 beim Schwimmen gesammelte Wasser nach unten ausströmen kann.

## Patentansprüche

1. Brustprothese zum Tragen im Oberteil eines Badebekleidungsstücks, die einen weich-elastischen, schalenförmigen Kunststoffkörper (1), der von einer Polyurethanfolie umhüllt ist, aufweist, wobei die dem Körper der Trägerin zugewandte Rückseite des Kunststoffkörpers (1) eine Aushöhlung (3) und eine einzige Versteifungsrippe aufweist (4b), die aus der Oberfläche der Aushöhlung vorspringt und sich geradlinig und ununterbrochen zwischen dem beim Tragen der Prothese oberen und unteren Rand der Aushöhlung (3) erstreckt.

2. Brustprothese nach Anspruch 1, dadurch gekennzeichnet, daß die Versteifungsrippe (4b) einen in Längsrichtung verlaufenden Rücken (5) aufweist, der am beim Tragen der Prothese oberen Ende der Versteifungsrippe (4b) tiefer als der beim Tragen der Prothese obere Rand (2) der Prothese ist und am beim Tragen der Prothese unteren Ende der Versteifungsrippe über den beim Tragen der Prothese unteren Rand (2) der Prothese in Richtung des Körpers der Trägerin vorspringt.

3. Brustprothese nach Anspruch 2, dadurch gekennzeichnet, daß der Rücken (5) der Versteifungsrippe (4) im mittleren Bereich der Versteifungsrippe (4b) tiefer als der beim Tragen der Prothese obere und seitliche Rand (2) der Prothese ist.

4. Brustprothese nach einem der vorhergehenden Ansprüche, dadurch gekennzeichnet, daß die Prothese bezüglich einer rechtwinklig auf ihrer projizierten Grundfläche stehenden Mittelebene (ME) symmetrisch ist, die beim Tragen der Prothese parallel zur Längsrichtung des Körpers der Trägerin verläuft, wobei die Versteifungsrippe (4b) auf der Mittelebene (ME) der Prothese verläuft.

5. Brustprothese nach Anspruch 4, dadurch gekennzeichnet, daß die Grundfläche annähernd die Form eines gleichschenkligen Dreiecks hat, dessen Ecken abgerundet sind und dessen Basis konvex gekrümmt ist und dessen Schenkel einen Winkel α von 70° bis 90° einschließen und die Versteifungsrippe (4b) auf der Höhenlinie des Dreiecks verläuft.

6. Brustprothese nach Anspruch 1, dadurch gekennzeichnet, daß die Versteifungsrippe einen in Längsrichtung der Rippe sich erstreckenden Rücken (5) hat, der in einem mittleren Längsabschnitt der Rippe (4b) konkav gebogen ist.

7. Brustprothese zum Tragen im Oberteil eines Badebekleidungsstücks, die einen weich-elastischen Kunststoffkörper (1), der von einer Polyurethanfolie umhüllt ist, aufweist, wobei die dem Körper der Trägerin zugewandte Rückseite des Kunststoffkörpers (1) eine Aushöhlung (3) und mehrere aus der Oberfläche der Aushöhlung vorspringende Versteifungsrippen (4a, 4b, 4c) aufweist, die parallel zueinander verlaufen und sich geradlinig und ununterbrochen zwischen dem beim Tragen der Prothese oberen und unteren Rand der Aushöhlung (3) erstrecken.

8. Brustprothese nach Anspruch 7, dadurch gekennzeichnet, daß die Prothese bezüglich einer rechtwinklig auf ihrer projizierten Grundfläche stehenden Mittelebene (ME) symmetrisch ist, die beim Tragen der Prothese parallel zur Längsrichtung des Körpers der Trägerin verläuft, wobei eine der Versteifungsrippen (4a, 4b, 4c) auf der Mittelebene (ME) der Prothese verläuft.

9. Brustprothese nach Anspruch 8, dadurch gekennzeichnet, daß drei parallele Versteifungsrippen (4a, 4b, 4c) vorgesehen sind, von denen die mittlere (4b) auf der Mittelebene (ME) der Prothese verläuft.

10. Brustprothese nach Anspruch 8 oder 9, dadurch gekennzeichnet, daß die Grundfläche annähernd die Form eines gleichschenkligen Dreiecks hat, dessen Ecken abgerundet sind und dessen Basis konvex gekrümmt ist und dessen Schenkel einen Winkel α von 70° bis 90° einschließen und eine der Versteifungsrippen (4a, 4b, 4c) auf der Höhenlinie des Dreiecks verläuft.

11. Brustprothese nach einem der Ansprüche 7 bis 10, dadurch gekennzeichnet, daß jede Versteifungsrippe (4a, 4b, 4c) einen in Längsrichtung verlaufenden Rücken (5) aufweist, der am beim Tragen der Prothese oberen Ende der jeweiligen Versteifungsrippe (4a, 4b, 4c) tiefer als der beim Tragen der Prothese obere Rand (2) der Prothese ist und am beim Tragen der Prothese unteren Ende der jeweiligen Versteifungsrippe (4a, 4b, 4c) über den beim Tragen der Prothese unteren Rand (2) der Prothese in Richtung des Körpers der Trägerin vorspringt.

12. Brustprothese nach Anspruch 11, dadurch gekennzeichnet, daß der Rücken (5) jeder Versteifungsrippe (4a, 4b, 4c) im mittleren Bereich der jeweiligen Versteifungsrippe (4a, 4b, 4c) tiefer als der beim Tragen der Prothese obere und seitliche Rand (2) der Prothese ist.

13. Brustprothese nach ein Anspruch 7, dadurch gekennzeichnet, daß jede Versteifungsrippe (4a, 4b, 4c) einen in Längsrichtung der Rippe sich erstreckenden Rücken (5) hat, der in einem mittleren Längsabschnitt der Rippe (4a, 4b, 4c) konkav gebogen ist.

## Claims

1. A breast prosthesis for wearing in the top of a bathing costume, comprising a soft-elastic dish-shaped plastic member (1) encased in a polyurethane sheet, the back of the plastic member (1) facing the wearer's body having a cavity (3) and a single reinforcing rib (4b), which projects from the surface of the cavity and extends in a straight line continuously between the top and bottom edge of the cavity (3) when the prosthesis is worn.

2. A breast prosthesis according to claim 1, characterised in that the reinforcing rib (4b) has a longitudinally extending spine (5) which, when the prosthesis is worn, is deeper at the top end of the reinforcing rib (4b) than the top end of the prosthesis when worn and which projects at the bottom end of the reinforcing rib when the prosthesis is worn, beyond the bottom edge (2) of the prosthesis when worn and in the direction of the wearer's body.

3. A breast prosthesis according to claim 2, characterised in that spine (5) on the reinforcing rib (4) is lower in the middle portion of the reinforcing rib (4) than the upper and side edge of the prosthesis when the prosthesis is worn.

4. A breast prosthesis according to any of the preceding claims, characterised in that the prosthesis is symmetrical with respect to a central plane (ME) at right angles to its projected base surface and extending parallel to the longitudinal direction of the wearer's body when the prosthesis is worn, the reinforcing rib (4b) extending along the central plane (ME) of the prosthesis.

5. A breast prosthesis according to claim 4, characterised in that the base surface has approximately the shape of an isosceles triangle, the apices of which are rounded and the base of which has a convex curvature, and the sides of which include an angle α of 70° to 90° and the reinforcing rib (4b) extends along the triangle height line.

6. A breast prosthesis according to claim 1, characterised in that the reinforcing rib has a spine (5) extending in the longitudinal direction of the rib and having a concave curvature in the central longitudinal portion of the rib (4b).

7. A breast prosthesis for wearing in the top of a swimming costume and comprising a soft elastic plastic member (1) encased in a polyurethane sheet, wherein the back of the plastic member (1) facing the wearer's body has a cavity (3) and a number of reinforcing ribs (4a, 4b, 4c) projecting from the surface of the cavity and running parallel to one another and extending in a straight line and continuously between the upper and lower edge of the recess (3) when the prosthesis is worn.

8. A breast prosthesis according to claim 7, characterised in that the prosthesis is symmetrical with respect to a central plane (ME) at right angles to its projected base surface and extending parallel to the longitudinal direction of the wearer's body when the prosthesis is worn, one of the reinforcing ribs (4a, 4b, 4c) extending along the central plane (ME) of the prosthesis.

9. A breast prosthesis according to claim 8, characterised in that three parallel reinforcing ribs (4a, 4b, 4c) are provided, the central rib (4b) extending along the central plane (ME) of the prosthesis.

10. A breast prosthesis according to claim 8 or 9, characterised in that the base surface has approximately the shape of an isosceles triangle whose apices are rounded and whose base has a convex curvature and the sides of which include an angle α of 70° to 90°C and one of the reinforcing ribs (4a, 4b, 4c) extends along the triangle height line.

11. A breast prosthesis according to any of claims 7 to 10, characterised in that each reinforcing rib (4a, 4b, 4c) has a longitudinally extending spine (5) which is lower at the top end of the respective rib (4a, 4b, 4c) when the prosthesis is worn than the top edge (2) of the prosthesis when the prosthesis is worn and, at the bottom end of the respective rib (4a, 4b, 4c) when the prosthesis is worn, projects beyond the bottom edge (2) of the prosthesis when the prosthesis is worn, in the direction of the wearer's body.

12. A breast prosthesis according to claim 11, characterised in that the spine (5) of each reinforcing rib (4a, 4b, 4c) is lower in the central region of the respective reinforcing rib (4a, 4b, 4c) than the upper and side edge (2) of the prosthesis when the prosthesis is worn.

13. A breast prosthesis according to claim 7, characterised in that each reinforcing rib (4a, 4b, 4c) has a spine (5) which extends in the longitudinal direction of the rib and has a concave curvature in a central longitudinal portion of the rib (4a, 4b, 4c).

## Revendications

1. Prothèse mammaire à porter dans la partie supérieure d'un maillot de bain, qui présente un corps de matière plastique (1) en forme de coque, souple et élastique, enrobé d'un film de polyuréthanne, la face arrière du corps de matière plastique (1), tournée vers le corps de la porteuse, présentant un creux (3) et une nervure de raidissement unique (4b), qui dépasse de la surface du creux et s'étend linéairement et d'un seul tenant entre le bord supérieur et inférieur du creux (3), lors du port de la prothèse.

2. Prothèse mammaire suivant la revendication 1, caractérisée en ce que la nervure de raidissement (4b) présente un dos (5) situé dans le sens longitudinal, plus profond sur l'extrémité supérieure de la nervure de raidissement (4b), lors du port de la prothèse, que le bord supérieur (2) de la prothèse, lors du port de cette dernière, et qui dépasse, sur l'extrémité inférieure de la nervure de raidissement lors du port de la prothèse, du bord inférieur (2) de la prothèse, lors du port de cette dernière, en direction du corps de la porteuse.

3. Prothèse mammaire suivant la revendication 2, caractérisée en ce que le dos (5) de la nervure de raidissement (4) est plus profond, dans la zone centrale de la nervure de raidissement (4b), que le bord supérieur et latéral (2) de la prothèse, lors du port de cette dernière.

4. Prothèse mammaire suivant l'une des revendications précédentes, caractérisée en ce que la prothèse est symétrique par rapport à un plan médian (ME), perpendiculaire à sa surface de base projetée et qui se situe, lors du port de la prothèse, parallèlement au sens longitudinal du corps de la porteuse, la nervure de raidissement (4b) se situant sur le plan médian (ME) de la prothèse.

5. Prothèse mammaire suivant la revendication 4, caractérisée en ce que la surface de base présente à peu près la forme d'un triangle isocèle, dont les angles sont arrondis, dont la base a une courbure convexe, et dont les côtés forment un angle α de 70 à 90°, la nervure de raidissement (4b) se situant sur la hauteur du triangle.

6. Prothèse mammaire suivant la revendication 1, caractérisée en ce que la nervure de raidissement présente un dos (5) s'étendant dans le sens longitudinal de la nervure et doté d'une courbure concave dans une section longitudinale centrale de la nervure (4b).

7. Prothèse mammaire à porter dans la partie supérieure d'un maillot de bain, qui présente un corps de matière plastique (1) souple et élastique, enrobé d'un film de polyuréthanne, la face arrière du corps de matière plastique (1), tournée vers le corps de la porteuse, présentant un creux (3) et plusieurs nervures de raidissement (4a, 4b, 4c), qui dépassent de la surface du creux, sont parallèles entre elles, et s'étendent linéairement et d'un seul tenant entre le bord supérieur et inférieur du creux (3), lors du port de la prothèse.

8. Prothèse mammaire suivant la revendication 7, caractérisée en ce que la prothèse est symétrique par rapport à un plan médian (ME), perpendiculaire à sa surface de base projetée et qui se situe, lors du port de la prothèse, parallèlement au sens longitudinal du corps de la porteuse, l'une des nervures de raidissement (4a, 4b, 4c) se situant sur le plan médian (ME) de la prothèse.

9. Prothèse mammaire suivant la revendication 8, caractérisée par trois nervures de raidissement parallèles (4a, 4b, 4c), dont la nervure centrale (4b) se situe sur le plan médian (ME) de la prothèse.

10. Prothèse mammaire suivant l'une des revendications 8 et 9, caractérisée en ce que la surface de base présente à peu près la forme d'un triangle isocèle, dont les angles sont arrondis, dont la base a une courbure convexe, et dont les côtés forment un angle a de 70° à 90°, l'une des nervures de raidissement (4a, 4b, 4c) se situant sur la hauteur du triangle.

11. Prothèse mammaire suivant l'une des revendications 7 à 10, caractérisée en ce que chaque nervure de raidissement (4a, 4b, 4c) présente un dos (5) situé dans le sens longitudinal , ce dos étant plus profond sur l'extrémité supérieure de la nervure de raidissement respective (4a, 4b, 4c), lors du port de la prothèse, que le bord supérieur (2) de la prothèse, lors du port de cette dernière, et dépassant, sur l'extrémité inférieure de la nervure de raidissement respective (4a, 4b, 4c), lors du port de la prothèse, du bord inférieur (2) de la prothèse, lors du port de cette dernière, en direction du corps de la porteuse.

12. Prothèse mammaire suivant la revendication 11, caractérisée en ce que le dos (5) de chaque nervure de raidissement (4a, 4b, 4c) est plus profond, dans la zone centrale de la nervure respective (4a, 4b, 4c), que le bord supérieur et latéral (2) de la prothèse, lors du port de cette dernière.

13. Prothèse mammaire suivant la revendication 7, caractérisée en ce que chaque nervure de raidissement (4a, 4b, 4c) présente un dos (5) s'étendant dans le sens longitudinal de la nervure, doté d'une courbure concave dans une section longitudinale centrale de la nervure (4a, 4b, 4c).
